# FASCICULE DE BREVET EUROPEEN

(11) **EP 2 791 097 B1**
(45) Date de publication et mention de la délivrance du brevet: **05.02.2020**
(21) Numéro de dépôt: 12816705.3
(22) Date de dépôt: 11.12.2012
(51) Int. Cl.: C07C 41/30, C07C 43/253

(54) **SYNTHESE A HAUTS RENDEMENTS DE P-(BENZYLOXY)CALIX[6,7,8]ARENES**
SYNTHESE VON P-(BENZYLOXY)CALIX[6,7,8]ARENEN MIT HOHEM ERTRAG
HIGH YIELD SYNTHESIS OF P-(BENZYLOXY)CALIX[6,7,8]ARENES

(30) Priorité: 12.12.2011 FR 1161497; 28.08.2012 FR 1258051
(43) Date de publication de la demande: 22.10.2014
(73) Titulaire: Université Paris-Sud XI, 91405 Orsay Cedex (FR)
(72) Inventeur: HUC, Vincent, Germain, 91400 Orsay (FR); MARTINI, Cyril, 91140 VILLEBON-SUR-YVETTE FRANCE (FR)
(74) Mandataire: Grosset-Fournier, Chantal Catherine
(86) Numéro de dépôt international: PCT/FR2012/052877
(87) Numéro de publication internationale: WO 2013/088056

(56) Documents cités:
- FR-A1- 2 797 442
- ALESSANDRO CASNATI ET AL: "p -(Benzyloxy)calix[8]arene: One-Pot Synthesis and Functionalization", THE JOURNAL OF ORGANIC CHEMISTRY, vol. 62, no. 18, 1 septembre 1997 (1997-09-01), pages 6236-6239, XP055034562, ISSN: 0022-3263, DOI: 10.1021/jo970620r
- GUTSCHE C D ET AL: "CALIXARENES. 4. THE SYNTHESIS, CHARACTERIZATION, AND PROPERTIES OF THE CALIXARENES FROM P-TERT-BUTYLPHENOL", JOURNAL OF THE AMERICAN CHEMICAL SOCIETY, ACS PUBLICATIONS, vol. 103, no. 13, 1 juillet 1981 (1981-07-01), pages 3782-3792, XP000676352, ISSN: 0002-7863, DOI: 10.1021/JA00403A028
- NAKAYAMA T ET AL: "NEW POSITIVE-TYPE PHOTORESIST BASED ON MONO-SUBSTITUTED HYDROQUINONE CALIX (8) ARENE AND DIAZONAPHTHOQUINONE", JOURNAL OF MATERIALS CHEMISTRY, THE ROYAL SOCIETY OF CHEMISTRY, CAMBRIDGE, GB, vol. 9, no. 3, 1 mars 1999 (1999-03-01), pages 697-702, XP000853243, ISSN: 0959-9428, DOI: 10.1039/A807718E
- MICHAEL A. MARKOWITZ ET AL: "Perforated monolayers: design and synthesis of porous and cohesive monolayers from mercurated calix[n]arenes", JOURNAL OF THE AMERICAN CHEMICAL SOCIETY, vol. 111, no. 21, 1 octobre 1989 (1989-10-01), pages 8192-8200, XP055034571, ISSN: 0002-7863, DOI: 10.1021/ja00203a020

## Description

La présente invention concerne la synthèse à hauts rendements de *p-*(benzyloxy)calix[6,7,8] arènes.

Les calixarènes ont été particulièrement étudiés ces dix dernières années en raison des possibilités immenses offertes par ces macrocycles aisément accessibles. Ces macrocycles possèdent une forme de calice dont la cavité est inférieure ou égale à environ 1 nm.

Quelques unes des propriétés les plus étudiées incluent les phénomènes de reconnaissance des ions et/ou des molécules, les assemblages supramoléculaires et la synthèse des nanoparticules.

Dans la plupart des cas, ces études ont été effectuées avec les calixarènes fonctionnalisés par un *p*-(*t*-butyle) étant donné que la synthèse de ces dérivés est de loin la plus documentée depuis le travail pionnier de Gutsche et al.

Gutsche et al., J. Am. Chem. Soc., 1981, vol.103, n°13, pp.3782-3792.

D. Gutsche, Calixarenes: An Introduction, The Royal Society of Chemistry, Cambridge, 2008**.**

(Z. Asfari, V. Böhmer, J. Harrowfield, J. Vicens (Eds.), Calixarenes 2001, Kluwer, Dordrecht, the Netherlands, 2001**.**

Par conséquent, l'utilisation d'autres phénols fonctionnalisés pour la synthèse des calixarènes est en grande partie inexplorée, même si des synthèses en une étape de *p-*(alkyls)calixarènes sont connues (T. Patrick, P. Egan, J. Org. Chem. 1977, 42, 382; T. Patrick, P. Egan, J. Org. Chem. 1978, 43, 4280; Z. Asfari, J. Vicens, Tetrahedron Lett. 1988, 29, 2659 ; F. Vocanson, M. Perrin, R. Lamartine, J. Inclusion Phenom. Macrocyclic Chem. 2001, 39, 127; Jerry L. Atwood et al; Org. Lett. 1999, 1, 1523).

Les calixarènes *p*-substitués sont habituellement obtenus en mélange de calix[4, 5, 6, 7, 8] par réaction d'un phénol *p*-substitué avec du paraformaldéhyde en présence d'au moins une base telle que la potasse ou la soude (B. Dahwan et al., Macromolec. Chem. 1987, 188, 921 ; C. D. Gutsche et al. Org. Synth. 1990, 68, 234; C. D. Gutsche et al. Org. Synth. 1990, 68, 238).

En fonction des conditions exactes utilisées (nature de la base alcaline utilisée comme catalyseur, température...) il est possible d'orienter la réaction pour conduire préférentiellement à une taille de cycle précise. En général, le *p*-(*t*-Bu)calix[8]arène est le calixarène obtenu le plus facilement, car il correspond au produit cinétique de la réaction de polycondensation.

Le *p*-(benzyloxy)calix[6] arène est un calixarène déjà décrit dans la littérature sous forme neutralisée mais obtenu soit en tant que produit très minoritaire de la synthèse du *p-*(benzyloxy)calix[8] arène, après plusieurs étapes de purifications avec un rendement de 1 à 2% (A. Casnati et al., J. Am. Chem. Soc. 2001, 123, 12182-12190), soit sous forme relativement impure avec une pureté de l'ordre de 80% (V. Huc et al., Eur. J. Org. Chem. 2010, 6186-6192).

Le *p*-(benzyloxy)calix[7] arène comporte dans sa structure 7 motifs de répétitions. Cette taille de cycle est habituellement considérée comme difficile à obtenir dans le domaine de la chimie des calixarènes. En effet, les études antérieures montrent que les calixarènes comportant un nombre impair de motifs sont difficilement accessibles et/ou plus difficiles à purifier. Dans le cas précis du *p*-(benzyloxy)calix[7] arène, ce produit est déjà décrit comme un sous produit très minoritaire de la synthèse d'autres calixarènes avec des rendements inférieurs à 10%, utilisant des procédures de purification complexes incluant des purifications par chromatographie sur colonne (V. Huc et al., Eur. J. Org. Chem, 2010, 6186-6192). D'autres calix[7]arènes sont également connus, notamment en série *p*-(*t*-Bu), mais là encore les rendements sont faibles (*p*-(*t-*Bu) : A. Ninegawa et al., Macromol. Chem. Rapid. Chem. 1982, 3, 65 ; Y. Nakamoto et al. Macromol. Chem. Rapid. Chem. 1982, 3, 705 ; F. Vocanson et al., New. J. Chem. 1995, 19, 825 ; *p*-(benzyl) : J. L. Atwood et al., Org. Lett. 1999, 1523).

Le *p*-(benzyloxy)calix[7] arène sous forme monosalifiée n'est pas décrit dans la littérature et son obtention ouvrirait la voie à de nouveaux calix[7]arènes fonctionnalisés, notamment sur le phénol salifié.

Le *p*-(benzyloxy)calix[8] arène est obtenu dans l'art antérieur avec un rendement de 48% par réaction de *p*-(benzyloxy)phénol en présence de formaldéhyde avec une base choisie parmi la soude, la potasse ou l'hydroxyde de lithium, la base étant à une concentration par rapport au *p-*(benzyloxy)phénol de 0,02 équivalent (A Casnati et al, J. Org. Chem. 1997, 62, 6236-6239).

Dans la plupart des cas, la post-fonctionnalisation de la position para de ces calixarènes (pas toujours possible) est au mieux délicate. Dans le cas le plus courant des *p*-(*t*-Bu) calixarènes, il s'agit en général d'un procédé en plusieurs étapes nécessitant l'utilisation de réactifs pour retirer le *tert*-butyle*.* La réaction n'étant pas quantitative, la deterbutylation devient problématique lorsque le nombre d'unités du calixarène augmente. La présence de sous-produits limite le rendement, impose une étape de purification et restreint la pureté du produit. Ceci peut limiter le rendement final en produit entièrement déprotégé.

Par conséquent, l'obtention de calixarènes aisément fonctionnalisables sur la couronne haute par une large variété de groupements chimiques dans des conditions douces est toujours une question ouverte et notamment l'obtention de *p*-(benzyloxy)calix[6] arène et de *p-*(benzyloxy)calix[7]arène aisément fonctionnalisables avec des rendements de plus de 50% et une purification aisée en une seule étape .

L'un des objets de l'invention est de fournir un procédé de synthèse permettant :
l'obtention de *p*-(benzyloxy)calix[6]arènes purs avec un rendement de plus de 50% et une procédure de purification en une étape par simple cristallisation ou,
l'obtention de *p*-(benzyloxy)calix[7]arène, avec un rendement de plus de 50% et une procédure de purification en une étape par simple filtration ou cristallisation sélective dans un mélange de solvants organiques ou,
l'obtention de *p*-(benzyloxy)calix[8]arène, avec un rendement de plus de 50%, ou
l'obtention d'un mélange des trois, ou d'un mélange de deux choisis parmi les trois.

Un autre objet de l'invention est de fournir le *p*-(benzyloxy)calix[7]arène sous forme de monosel de césium.

Décrite dans la présente demande est l'utilisation de *p*-(benzyloxy)calix[6]arène ou de *p-*(benzyloxy)calix[7] arène ou d'un mélange des deux, ou d'un mélange de *p-*(benzyloxy)calix[6]arène ou de *p*-(benzyloxy)calix[7]arène pour la constitution d'un matériau.

La présente décrit l'utilisation d'au moins une base, en particulier choisie parmi l'hydroxyde de lithium, la potasse, la soude, l'hydroxyde de rubidium ou l'hydroxyde de césium, avec du p-(benzyloxy)phénol, ladite base étant à une concentration totale comprise de 0,09 équivalent à 0,5 équivalent par rapport audit p-(benzyloxy)phénol, pour la préparation d'un mélange comprenant du *p*-(benzyloxy)calix[6]arène, du *p*-(benzyloxy)calix[7]arène et du *p-*(benzyloxy)calix[8]arène, ou d'un mélange comprenant du *p*-(benzyloxy)calix[6]arène et du *p-*(benzyloxy)calix[7]arène, ou d'un mélange comprenant du *p*-(benzyloxy)calix[7]arène et du *p-*(benzyloxy)calix[8]arène, ou d'un mélange comprenant du *p*-(benzyloxy)calix[6]arène et du *p-*(benzyloxy)calix[8]arène, ou d'un composé consistant en du *p*-(benzyloxy)calix[6]arène, *du p-*(benzyloxy)calix[7]arène ou du *p*-(benzyloxy)calix[8]arène.

Le *p*-(benzyloxy)calix[6]arène possède la structure suivante :

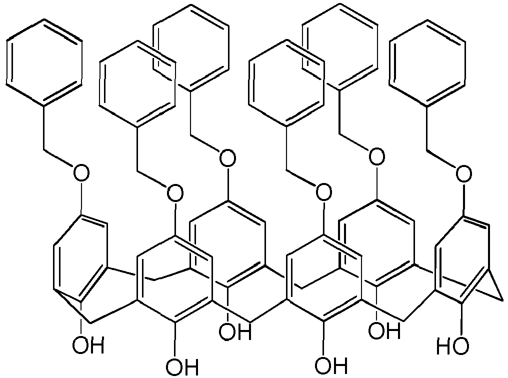

Le p-(benzyloxy)calix[7]arène possède la structure suivante :

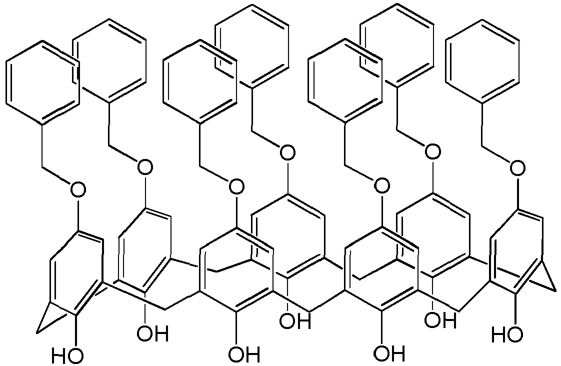

Le p-(benzyloxy)calix[8]arène possède la structure suivante :

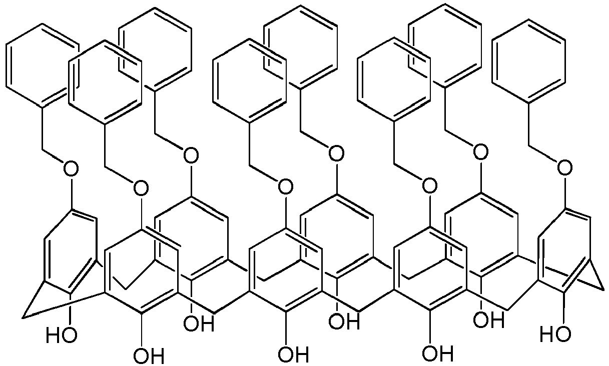

La présente concerne l'utilisation de l'hydroxyde de césium, avec du *p*-(benzyloxy)phénol, ladite base étant à une concentration totale comprise de 0,09 équivalent à 0,5 équivalent par rapport audit *p*-(benzyloxy)phénol, pour la préparation d'un mélange comprenant du *p*-(benzyloxy)calix[6]arène et du *p*-(benzyloxy)calix[7]arène, ou d'un composé consistant en du *p*-(benzyloxy)calix[6]arène ou du *p*-(benzyloxy)calix[7]arène.

Les Inventeurs ont trouvés de manière surprenante que la combinaison d'au moins une base telle que l'hydroxyde de césium et d'une concentration totale comprise de 0,09 équivalent à 0,5 équivalent par rapport audit *p*-(benzyloxy)phénol permettait d'obtenir le *p-*(benzyloxy)calix[6]arène et le *p*-(benzyloxy)calix[7] arène à plus de 50%.

Dans un mode de réalisation avantageux, ledit mélange comprenant du *p-*(benzyloxy)calix[6]arène et du *p*-(benzyloxy)calix[7]arène, obtenu avec l'hydroxyde de césium à une concentration totale comprise de 0,09 équivalent à 0,5 équivalent et du p-(benzyloxy)phénol, est majoritaire, c'est-à-dire est obtenu en proportion de plus de 50% (déterminée par RMN),
et un mélange de *p*-(benzyloxy)calix[9-20]arènes en proportion minoritaire est également obtenu.

Par mélange de *p*-(benzyloxy)calix[9-20]arènes, il faut comprendre un mélange de *p-*(benzyloxy)calix[9]arène, *p*-(benzyloxy)calix[10]arène, *p*-(benzyloxy)calix[11]arène, *p-*(benzyloxy)calix[12]arène, *p*-(benzyloxy)calix[13]arène, *p*-(benzyloxy)calix[14]arène, *p-*(benzyloxy)calix[15]arène, *p*-(benzyloxy)calix[16]arène, *p*-(benzyloxy)calix[17]arène, *p-*(benzyloxy)calix[18]arène, *p*-(benzyloxy)calix[19]arène, *p*-(benzyloxy)calix[9-20]arène, chacun d'eux pouvant être présent en proportion de 0 à 100% en poids, à condition qu'au moins un d'entre eux soit présent à une proportion différente de 0% et que la somme du pourcentage de chacun d'entre-eux soit égale à 100%, dans ledit mélange de *p*-(benzyloxy)calix[9-20]arènes.

Les *p*-(benzyloxy)calix[9-20]arènes sont également dénommés grands calixarènes dans la description.

La présente décrit l'utilisation d'au moins une base, en particulier l'hydroxyde de rubidium, avec du *p*-(benzyloxy)phénol, ladite base étant à une concentration totale comprise de 0,09 équivalent à 0,5 équivalent par rapport audit *p*-(benzyloxy)phénol, pour la préparation d'un mélange comprenant du *p*-(benzyloxy)calix[6]arène, du *p*-(benzyloxy)calix[7] arène et du *p-*(benzyloxy)calix[8]arène et un mélange de *p*-(benzyloxy)calix[9-20]arènes.

La présente décrit l'utilisation d'au moins une base, en particulier l'hydroxyde de rubidium, avec du *p*-(benzyloxy)phénol, ladite base étant à une concentration totale comprise de 0,09 équivalent à 0,3 équivalent par rapport audit *p*-(benzyloxy)phénol, pour la préparation d'un mélange comprenant du *p*-(benzyloxy)calix[6]arène, du *p*-(benzyloxy)calix[7]arène et du *p-*(benzyloxy)calix[8]arène et un mélange de *p*-(benzyloxy)calix[9-20]arènes.

Les Inventeurs ont trouvé que la combinaison d'au moins une base telle que l'hydroxyde de rubidium et d'une concentration totale comprise de 0,09 équivalent à 0,3 équivalent par rapport audit *p*-(benzyloxy)phénol permettait d'obtenir un mélange de *p-*(benzyloxy)calix[6]arène et de *p*-(benzyloxy)calix[7]arène à 50% ou plus, ainsi que le mélange de *p*-(benzyloxy)calix[9-20]arènes.

La présente décrit l'utilisation d'au moins une base, en particulier choisie parmi la potasse, la soude, avec du *p*-(benzyloxy)phénol, ladite au moins une base étant à une concentration totale comprise de 0,09 équivalent à 0,5 équivalent par rapport audit *p-*(benzyloxy)phénol, pour la préparation d'un mélange comprenant du *p*-(benzyloxy)calix[6]arène, du *p*-(benzyloxy)calix[7]arène et du *p*-(benzyloxy)calix[8]arène, ou d'un mélange comprenant du *p*-(benzyloxy)calix[7]arène et du *p*-(benzyloxy)calix[8]arène, ou d'un mélange comprenant du *p-*(benzyloxy)calix[6]arène et du *p*-(benzyloxy)calix[8]arène, ou d'un mélange comprenant *du p-*(benzyloxy)calix[6]arène et du *p*-(benzyloxy)calix[7]arène, ou d'un composé consistant en du *p-*(benzyloxy)calix[6]arène, du *p*-(benzyloxy)calix[7]arène ou du *p*-(benzyloxy)calix[8]arène.

Les inventeurs ont trouvé que la combinaison d'au moins une base telle que la potasse et/ou la soude et d'une concentration totale comprise de 0,09 équivalent à 0,5 équivalent par rapport audit *p*-(benzyloxy)phénol permettait d'obtenir le *p-*(benzyloxy)calix[6]arène ou le *p*-(benzyloxy)calix[7]arène ou le *p*-(benzyloxy)calix[8]arène de façon majoritaire, ou un mélange de ceux-ci ou d'un mélange de deux parmi les trois, en fonction de la concentration totale en base introduite, avec de bons rendements.

L'utilisation de KOH ou de NaOH à concentration inférieure à 0,09 équivalent (par ex. 0,03 équivalent) conduit essentiellement à du *p*-(benzyloxy)calix[8]arène.

Un des avantages est donc la fourniture de *p*-(benzyloxy)calix[6]arène ou de *p*-(benzyloxy)calix[7]arène ou *p*-(benzyloxy)calix[8]arène ou d'un mélange de ceux-ci, dont les fonctions benzyloxy sont déprotégées dans des conditions très douces, par simple débenzylation par des techniques bien connues de l'homme du métier, permettant ainsi l'accès à des molécules qui pourront être aisément fonctionnalisées par la suite.

La présente invention concerne un procédé de préparation d'un composé consistant en un mélange comprenant du *p*-(benzyloxy)calix[6]arène et du *p-*(benzyloxy)calix[7]arène, ou d'un composé consistant en du *p*-(benzyloxy)calix[6]arène ou du *p-*(benzyloxy)calix[7]arène, comprenant une étape de mise en contact d'hydroxyde de césium avec du *p*-(benzyloxy)phénol et du paraformaldéhyde, ledit hydroxyde de césium étant à une concentration totale comprise de 0,09 équivalent à 0,5 équivalent par rapport audit *p*-(benzyloxy)phénol.

Selon un autre aspect, la présente invention concerne un procédé de préparation d'un composé consistant en un mélange comprenant du *p*-(benzyloxy)calix[9-20]arènes, du *p-*(benzyloxy)calix[6]arène, du *p*-(benzyloxy)calix[7]arène, et du *p*-(benzyloxy)calix[8]arène comprenant une étape de mise en contact d'hydroxyde de rubidium avec du *p*-(benzyloxy)phénol et du paraformaldéhyde, ladite une base étant à une concentration totale comprise de 0,09 équivalent à 0,5 équivalent par rapport audit *p*-(benzyloxy)phénol.

La présente décrit aussi un procédé de préparation d'un composé consistant en un mélange comprenant du *p*-(benzyloxy)calix[9-20]arènes, du *p-*(benzyloxy)calix[6]arène, du *p*-(benzyloxy)calix[7]arène, et du *p*-(benzyloxy)calix[8]arène comprenant une étape de mise en contact d'hydroxyde de rubidium avec du *p*-(benzyloxy)phénol et du paraformaldéhyde, ladite une base étant à une concentration totale comprise de 0,09 équivalent à 0,3 équivalent par rapport audit *p*-(benzyloxy)phénol.

La présente décrit un procédé de préparation d'un composé consistant en un mélange comprenant du p-(benzyloxy)calix[6]arène, du *p-*(benzyloxy)calix[7]arène et du *p*-(benzyloxy)calix[8]arène, ou d'un composé consistant en du p-(benzyloxy)calix[6]arène, du *p*-(benzyloxy)calix[7]arène ou du *p*-(benzyloxy)calix[8]arène, comprenant la mise en contact d'au moins une base choisie parmi la soude ou la potasse avec du *p*-(benzyloxy)phénol et du paraformaldéhyde, ladite base étant à une concentration totale comprise de 0,09 équivalent à 0,5 équivalent par rapport audit *p*-(benzyloxy)phénol.

Selon un autre aspect la présente décrit un procédé de préparation d'un composé consistant en un mélange comprenant du *p*-(benzyloxy)calix[6]arène, du *p-*(benzyloxy)calix[7]arène et du *p*-(benzyloxy)calix[8]arène, ou un mélange comprenant du *p-*(benzyloxy)calix[6]arène et du *p*-(benzyloxy)calix[8]arène, ou d'un composé consistant en du p-(benzyloxy)calix[6]arène, du *p*-(benzyloxy)calix[7]arène ou du *p*-(benzyloxy)calix[8]arène, comprenant la mise en contact d'au moins une base choisie parmi la soude ou la potasse avec du *p*-(benzyloxy)phénol et du paraformaldéhyde, ladite base étant à une concentration totale comprise de 0,09 équivalent à 0,2 équivalent, en particulier 0,15 équivalent, par rapport audit *p-*(benzyloxy)phénol.

La présente décrit en outre un procédé de préparation d'un composé consistant en un mélange comprenant du *p*-(benzyloxy)calix[6]arène, du *p*-(benzyloxy)calix[7] arène et du *p*-(benzyloxy)calix[8]arène, ou un mélange comprenant du *p*-(benzyloxy)calix[6]arène et du *p*-(benzyloxy)calix[8]arène, ou d'un composé consistant en du *p*-(benzyloxy)calix[7]arène, comprenant la mise en contact d'au moins une base choisie parmi la soude ou la potasse avec du *p*-(benzyloxy)phénol et du paraformaldéhyde, ladite base étant à une concentration totale comprise de 0,09 équivalent à 0,2 équivalent, en particulier 0,15 équivalent, par rapport audit *p*-(benzyloxy)phénol, tel que défini ci-dessus, pour obtenir un mélange de *p-*(benzyloxy)calix[6]arène, de *p*-(benzyloxy)calix[7]arène et de *p*-(benzyloxy)calix[8]arène,
comprenant une étape additionnelle de filtration du mélange comprenant du *p-*(benzyloxy)calix[6]arène, du *p*-(benzyloxy)calix[7]arène et du *p*-(benzyloxy)calix[8]arène pour obtenir d'une part un précipité comprenant un composé consistant en du *p-*(benzyloxy)calix[7]arène sous forme neutralisée et d'autre part un filtrat comprenant un mélange de différents composés comprenant le *p*-(benzyloxy)calix[6]arène.

Par l'expression « sous forme neutralisée », il faut comprendre un calixarène dont tous les groupements phénols libres ont été neutralisés, c'est-à-dire sont sous forme OH, non salifiée.

Un avantage est donc de pouvoir obtenir aisément le *p-*(benzyloxy)calix[7]arène sous forme neutralisée par simple filtration du milieu réactionnel.

Les inventeurs ont trouvé que la combinaison d'au moins une base telle que la soude ou la potasse à une concentration totale comprise de 0,09 équivalent à 0,2 équivalent, par rapport audit *p*-(benzyloxy)phénol permettait d'obtenir un mélange comprenant du *p*-(benzyloxy)calix[6]arène, du *p*-(benzyloxy)calix[7]arène et du *p*-(benzyloxy)calix[8]arène dont le *p*-(benzyloxy)calix[7]arène sous forme neutralisée peut être séparé et obtenu pur par simple filtration.

Dans toute la description, le terme « pur » désigne un composé ayant une pureté supérieure à 95 %.

Selon un autre aspect la présente décrit un procédé de préparation d'un composé consistant en un mélange comprenant du *p*-(benzyloxy)calix[6] arène, du *p-*(benzyloxy)calix[7]arène et du *p*-(benzyloxy)calix[8]arène, ou un mélange comprenant du *p-*(benzyloxy)calix[6]arène et du *p*-(benzyloxy)calix[8]arène, ou d'un composé consistant en du *p-*(benzyloxy)calix[6]arène, du *p*-(benzyloxy)calix[7]arène ou du *p*-(benzyloxy)calix[8]arène, comprenant la mise en contact d'au moins une base choisie parmi la soude ou la potasse avec du *p*-(benzyloxy)phénol et du paraformaldéhyde, ladite base étant à une concentration totale supérieure à 0,2 équivalent et inférieure ou égale à 0,5 équivalent, en particulier de 0,3 équivalent, par rapport audit *p*-(benzyloxy)phénol.

La présente décrit aussi un procédé de préparation d'un composé consistant en un mélange comprenant du *p*-(benzyloxy)calix[6]arène, et du *p*-(benzyloxy)calix[7]arène, ou d'un composé consistant en du *p*-(benzyloxy)calix[8]arène, comprenant la mise en contact d'au moins une base choisie parmi la soude ou la potasse avec du *p*-(benzyloxy)phénol et du paraformaldéhyde, ladite base étant à une concentration totale supérieure à 0,2 équivalent et inférieure ou égale à 0,5 équivalent, en particulier de 0,3 équivalent, par rapport audit *p*-(benzyloxy)phénol tel que défini ci-dessus, pour obtenir un mélange de *p*-(benzyloxy)calix[6]arène, de *p*-(benzyloxy)calix[7]arène et de *p-*(benzyloxy)calix[8] arène, comprenant une étape additionnelle de filtration du mélange comprenant du *p*-(benzyloxy)calix[6]arène, du *p*-(benzyloxy)calix[7]arène et du *p-*(benzyloxy)calix[8]arène pour obtenir d'une part un précipité comprenant un composé consistant en du *p*-(benzyloxy)calix[8]arène pur sous forme neutralisée et d'autre part un filtrat comprenant un mélange comprenant le *p*-(benzyloxy)calix[6]arène et le *p*-(benzyloxy)calix[7]arène sous forme neutralisée.

Par l'expression « sous forme neutralisé », il faut comprendre un calixarène dont tous les groupements phénol ont été neutralisés, c'est-à-dire sont sous forme OH, non salifiée.

On peut ainsi obtenir aisément le *p-*(benzyloxy)calix[8]arène sous forme neutralisée par simple filtration du milieu réactionnel.

Le filtrat permet de récupérer le mélange de *p*-(benzyloxy)calix[6]arène et du *p-*(benzyloxy)calix[7]arène sous forme neutralisée.

Les inventeurs ont trouvé que la combinaison d'au moins une base telle que la soude ou la potasse à une concentration totale supérieure à 0,2 équivalent et inférieure ou égale à 0,5 équivalent par rapport audit *p*-(benzyloxy)phénol permettait d'obtenir un mélange comprenant du *p*-(benzyloxy)calix[6]arène, du *p*-(benzyloxy)calix[7]arène et du *p-*(benzyloxy)calix[8]arène dont le *p*-(benzyloxy)calix[8]arène sous forme neutralisée peut être séparé et obtenu pur par simple filtration.

La présente décrit un procédé de préparation d'un composé consistant en du *p*-(benzyloxy)calix[6]arène, ou du *p-*(benzyloxy)calix[7]arène, comprenant la mise en contact d'au moins une base choisie parmi la soude ou la potasse avec du *p*-(benzyloxy)phénol et du paraformaldéhyde, ladite base étant à une concentration totale supérieure à 0,2 équivalent et inférieure ou égale à 0,5 équivalent, en particulier de 0,3 équivalent, par rapport audit *p*-(benzyloxy)phénol et une étape additionnelle de filtration du mélange comprenant du *p*-(benzyloxy)calix[6]arène, du *p*-(benzyloxy)calix[7]arène et du *p*-(benzyloxy)calix[8]arène pour obtenir d'une part un précipité comprenant le *p-*(benzyloxy)calix[8]arène sous forme neutralisée et d'autre part un filtrat comprenant un mélange comprenant le *p*-(benzyloxy)calix[6]arène et le *p*-(benzyloxy)calix[7]arène sous forme neutralisée, tel que défini ci-dessus et,
comprenant une étape additionnelle de traitement du filtrat contenant le mélange comprenant du *p*-(benzyloxy)calix[6]arène et du *p*-(benzyloxy)calix[7]arène par un mélange de solvants comprenant du DMSO, pour obtenir d'une part un précipité consistant en du *p-*(benzyloxy)calix[6]arène et d'autre part un filtrat contenant le *p*-(benzyloxy)calix[7]arène sous forme neutralisée.

Les inventeurs ont trouvé que le traitement du filtrat ci-dessus, obtenu après filtration du *p*-(benzyloxy)calix[8]arène, par un mélange de solvant comprenant du DMSO, permettait de séparer le *p*-(benzyloxy)calix[6]arène, du *p*-(benzyloxy)calix[7]arène et d'obtenir ainsi notamment le *p*-(benzyloxy)calix[7]arène pur avec un rendement supérieur à 50%.

Le DMSO est mélangé à un solvant non polaire, tel que le toluène dans une proportion de 1 à 10 en volume.

Par solvant non polaire, il faut comprendre un solvant possédant un moment dipolaire faible ou nul, tel que, sans être limité à ceux-ci, le benzène, le toluène, le xylène, le cyclohexane, l'hexane, le pentane, l'éther de pétrole, le tétrachlorure de carbone...

Dans un mode de réalisation avantageux, la présente invention concerne un procédé de préparation d'un composé consistant en un mélange comprenant du *p*-(benzyloxy)calix[6]arène et du *p*-(benzyloxy)calix[7] arène, ou d'un composé consistant en du *p*-(benzyloxy)calix[6]arène ou du *p*-(benzyloxy)calix[7]arène comprenant une étape de mise en contact d'hydroxyde de césium avec du *p*-(benzyloxy)phénol et du paraformaldéhyde, dans lequel ladite base est à une concentration totale comprise de 0,09 équivalent à 0,5 équivalent pour obtenir un mélange comprenant du *p*-(benzyloxy)calix[6]arène et du *p*-(benzyloxy)calix[7] arène, et comprenant une étape additionnelle de neutralisation pour obtenir un mélange comprenant du *p-*(benzyloxy)calix[6]arène et du *p*-(benzyloxy)calix[7]arène sous forme neutralisée.

Les calixarènes obtenus dans le milieu réactionnel sont dans un environnement basique et nécessitent donc une étape de neutralisation, c'est-à-dire de traitement avec un acide pour les obtenir sous forme neutralisée.

Dans un mode de réalisation avantageux, la présente invention concerne un procédé de préparation d'un composé consistant en un mélange comprenant du *p*-(benzyloxy)calix[6]arène et du *p*-(benzyloxy)calix[7] arène, ou d'un composé consistant en du *p*-(benzyloxy)calix[6]arène ou du *p*-(benzyloxy)calix[7]arène, comprenant une étape de mise en contact d'hydroxyde de césium avec du *p*-(benzyloxy)phénol et du paraformaldéhyde, dans lequel ladite base est à une concentration totale comprise de 0,09 à 0,2 équivalent, en particulier 0,15 équivalent, pour obtenir un mélange comprenant du *p*-(benzyloxy)calix[6]arène et du *p*-(benzyloxy)calix[7]arène sous forme salifiée.

Les inventeurs ont trouvé de façon surprenante que le contrôle de la quantité de base telle que l'hydroxyde de césium, c'est-à-dire comprise de 0,09 à 0,2 équivalent par rapport au p-(benzyloxy)phenol, permettait de contrôler la réaction et de favoriser la formation de *p-*(benzyloxy)calix[7]arène par rapport au *p*-(benzyloxy)calix[6]arène conduisant donc à un mélange comprenant majoritairement du *p*-(benzyloxy)calix[7]arène.

Avantageusement, la concentration en césium est de 0,10 équivalent ou 0,11 équivalent ou 0,12 équivalent ou 0,13 équivalent ou 0,14 équivalent ou 0,15 équivalent ou 0,16 équivalent ou 0,17 équivalent ou 0,18 équivalent ou 0,19 équivalent ou 0,20 équivalent.

Plus avantageusement, la concentration en césium est de 0,15 équivalent.

Dans un mode de réalisation avantageux, le mélange comprenant du *p-*(benzyloxy)calix[6]arène et du *p*-(benzyloxy)calix[7]arène obtenu par le procédé dans lequel l'hydroxyde de césium est à une concentration totale comprise de 0,09 à 0,2 équivalent, en particulier 0,15 équivalent, comprend de 50 à 80% en poids de *p*-(benzyloxy)calix[7]arène, en particulier 70%, et de 20 à 50 % en poids de *p*-(benzyloxy)calix[6]arène.

Les pourcentages indiqués ici sont déterminés par RMN.

Dans un mode de réalisation avantageux, la présente invention concerne un procédé de préparation d'un composé consistant en un mélange comprenant du *p*-(benzyloxy)calix[6]arène et du *p*-(benzyloxy)calix[7]arène, un composé consistant en du *p*-(benzyloxy)calix[6]arène ou du *p-*(benzyloxy)calix[7]arène, comprenant une étape de mise en contact d'au moins une base choisie parmi l'hydroxyde de césium avec du *p*-(benzyloxy)phénol et du paraformaldéhyde, dans lequel ladite base est à une concentration totale comprise de 0,09 à 0,2 équivalent, en particulier 0,15 équivalent, pour obtenir un mélange comprenant du *p*-(benzyloxy)calix[6]arène et du *p-*(benzyloxy)calix[7]arène sous forme salifiée, et comprenant une étape additionnelle de filtration du mélange comprenant du *p*-(benzyloxy)calix[6] arène et du *p*-(benzyloxy)calix[7]arène sous forme salifiée pour obtenir le *p*-(benzyloxy)calix[7]arène sous forme de monosel de césium suivie d'une étape additionnelle de neutralisation du *p*-(benzyloxy)calix[7]arène sous forme de monosel de césium pour obtenir le *p*-(benzyloxy)calix[7]arène sous forme neutralisée.

De façon encore plus inattendue, les inventeurs ont trouvé que le *p-*(benzyloxy)calix[7]arène qui est sous forme salifiée, c'est-à-dire qu'un phénol présent sur la molécule est sous forme de phénolate de césium, peut être isolé majoritairement sous forme de monosel de césium.

Il faut noter que l'obtention du monosel de césium isolé est d'autant plus inattendue que le procédé faisant intervenir comme base de la soude ou de la potasse à 0,15 équivalent, après filtration, ne conduit pas à la précipitation du *p*-(benzyloxy)calix[7]arène sous forme salifiée.

Ceci peut s'expliquer par le fait que le césium est un alcalin dit mou, le phénolate de césium peut être stabilisé par des interactions avec les systèmes pi-conjugués des fonctions phényles présentes sur le calixarène permettant ainsi d'isoler le produit le plus stable d'un point de vue thermodynamique. De telles interactions sont beaucoup plus faibles avec les alcalins plus petits.

Une autre explication pourrait être une solubilité plus faible du monosel de césium par rapport aux autres sels alcalins. Cette faible solubilité pourrait induire une précipitation de ce monosel hors du milieu réactionnel au fur et à mesure de sa formation et ainsi déplacer les différents équilibres.

Un autre avantage de l'invention est que la mise à disposition de *p-*(benzyloxy)calix[7]arène sous forme de sel de césium permet la fonctionnalisation de la fonction phénolate de césium par un composé électrophile avant la déprotection des groupes benzyloxy permettant ainsi l'accès à des molécules fonctionnalisées différentes de celles obtenues ci-dessus.

Le procédé développé par les Inventeurs permet, outre l'obtention du monosel de césium, une augmentation substantielle du rendement de la réaction en *p*-(benzyloxy)calix[7] arène qui est supérieur ou égal à 50% contrairement à l'art antérieur qui ne dépasse pas 10%.

Dans un mode de réalisation avantageux, la présente invention concerne un procédé de préparation d'un composé consistant en un mélange comprenant du *p*-(benzyloxy)calix[6]arène et du *p*-(benzyloxy)calix[7] arène défini ci-dessus, comprenant les étapes suivantes :
a. mise en contact de l'hydroxyde de césium à une concentration de 0,09 à 0,2 équivalent, en particulier 0,15 équivalent, avec du *p*-(benzyloxy)phénol et du paraformaldéhyde dans un solvant puis chauffage, pour obtenir un mélange comprenant du *p*-(benzyloxy)calix[6]arène et du *p*-(benzyloxy)calix[7]arène sous forme salifiée,
b. neutralisation du mélange comprenant du *p*-(benzyloxy)calix[6]arène et du p-(benzyloxy)calix[7]arène sous forme salifiée avec un acide, pour obtenir un mélange comprenant du *p*-(benzyloxy)calix[6]arène et du *p-*(benzyloxy)calix[7]arène sous forme neutralisée.

Le solvant de l'étape a. est un solvant possédant un point d'ébullition élevé compris d'environ 100 à environ 200°C, en particulier environ 140°C, par exemple le xylène, mais sans être limité à celui-ci.

Le chauffage dépend donc de la nature du solvant et peut être effectué de 30°C à 200°C.

L'étape a. conduit à un mélange de *p*-(benzyloxy)calix[6]arène et du *p-*(benzyloxy)calix[7]arène sous forme salifiée.

La neutralisation du mélange est effectuée par un acide, par exemple de l'acide chlorhydrique aqueux ou de l'acide sulfurique aqueux.

Dans ce mode de réalisation, il est donc obtenu un mélange de *p*-(benzyloxy)calix[6]arène et du *p*-(benzyloxy)calix[7]arène sous forme neutralisée.

Dans un mode de réalisation avantageux, la présente invention concerne un procédé de préparation d'un composé consistant en un mélange comprenant du *p*-(benzyloxy)calix[6]arène et du *p*-(benzyloxy)calix[7]arène, ou d'un composé consistant en du *p*-(benzyloxy)calix[6]arène ou du *p*-(benzyloxy)calix[7]arène, comprenant les étapes suivantes :
a. mise en contact de l'hydroxyde de césium à une concentration de 0,09 à 0,2 équivalent, en particulier 0,15 équivalent, avec du *p*-(benzyloxy)phénol et du paraformaldéhyde, dans un solvant puis chauffage, pour obtenir un mélange comprenant du *p*-(benzyloxy)calix[6]arène et du *p*-(benzyloxy)calix[7]arène sous forme salifiée,
b. filtration dudit mélange comprenant du *p*-(benzyloxy)calix[6]arène et du p-(benzyloxy)calix[7] arène sous forme salifiée pour obtenir le *p-*(benzyloxy)calix[7]arène sous forme de monosel de césium,
c. optionnellement neutralisation du *p*-(benzyloxy)calix[7]arène sous forme de monosel de césium avec un acide pour obtenir le *p*-(benzyloxy)calix[7]arène sous forme neutralisée.

L'étape a. conduit à un mélange de *p*-(benzyloxy)calix[6]arène et du *p-*(benzyloxy)calix[7]arène sous forme salifiée mais seul le *p*-(benzyloxy)calix[7]arène sous forme de sel de césium précipite ce qui permet de l'isoler pur par simple filtration à l'étape b..

Le *p*-(benzyloxy)calix[7]arène peut alors être utilisé tel quel pour fonctionnalisation de la fonction phénolate de césium, ou fabrication d'un matériau ou alors neutralisé pour une fonctionnalisation des fonctions phénols libérées après débenzylation.

Dans un mode de réalisation avantageux, la présente invention concerne un procédé de préparation d'un composé consistant en un mélange comprenant du *p*-(benzyloxy)calix[6]arène et du *p*-(benzyloxy)calix[7]arène, ou d'un composé consistant en du *p*-(benzyloxy)calix[6]arène ou du *p*-(benzyloxy)calix[7]arène, comprenant une étape de mise en contact d'hydroxyde de césium à une concentration de 0,09 à 0,2 équivalent, en particulier 0,15 équivalent, pour obtenir un mélange comprenant du *p*-(benzyloxy)calix[6]arène et du *p*-(benzyloxy)calix[7]arène sous forme salifiée, tel que défini ci-dessus,
dans lequel le chauffage est effectué au reflux du solvant notamment du xylène.

Dans un mode de réalisation avantageux, la présente invention concerne un procédé de préparation d'un composé consistant en un mélange comprenant du *p*-(benzyloxy)calix[6]arène, du *p*-(benzyloxy)calix[7]arène, ou d'un composé consistant en du *p*-(benzyloxy)calix[6]arène ou du *p*-(benzyloxy)calix[7]arène, comprenant une étape de mise en contact d'hydroxyde de césium avec du *p*-(benzyloxy)phénol et du paraformaldéhyde, dans lequel ladite base est à une concentration totale supérieure à 0,2 équivalent et inférieure ou égale à 0,5 équivalent, en particulier de 0,3 équivalent, pour obtenir un mélange comprenant du *p*-(benzyloxy)calix[6]arène et du *p*-(benzyloxy)calix[7]arène sous forme salifiée.

De façon tout à fait inattendue, les Inventeurs ont trouvé que l'utilisation d'hydroxyde de césium à une concentration totale supérieure à 0,2 équivalent et inférieure ou égale à 0,5 équivalent permettait l'obtention d'un mélange comprenant du *p*-(benzyloxy)calix[6]arène et du *p*-(benzyloxy)calix[7]arène sous forme salifiée dans lequel le *p*-(benzyloxy)calix[6]arène est majoritaire et le *p*-(benzyloxy)calix[7]arène sous forme salifiée ne précipite pas, contrairement au procédé défini ci-dessus dans lequel l'hydroxyde de césium est utilisé à une concentration totale comprise de 0,09 à 0,2 équivalent.

Dans un mode de réalisation avantageux, la concentration totale d'hydroxyde de césium est de 0,21 équivalent, ou de 0,22 équivalent, ou de 0,23 équivalent, ou de 0,24 équivalent, ou de 0,25 équivalent, ou de 0,26 équivalent, ou de 0,27 équivalent, ou de 0,28 équivalent, ou de 0,29 équivalent.

Dans un mode de réalisation avantageux, le mélange comprenant du *p-*(benzyloxy)calix[6]arène et du *p*-(benzyloxy)calix[7]arène obtenu par le procédé dans lequel l'hydroxyde de césium est à une concentration totale supérieure à 0,2 équivalent et inférieure ou égale à 0,5 équivalent, en particulier de 0,3 équivalent, comprend de 50 à 80% en poids de *p-*(benzyloxy)calix[6]arène et 10 à 20% en poids de *p*-(benzyloxy)calix[7]arène.

Les pourcentages indiqués ici sont déterminés par RMN.

Dans un mode de réalisation avantageux, la présente invention concerne un procédé de préparation d'un composé consistant en un mélange comprenant du *p*-(benzyloxy)calix[6]arène et du *p*-(benzyloxy)calix[7]arène, ou d'un composé consistant en du *p*-(benzyloxy)calix[6]arène ou du *p*-(benzyloxy)calix[7]arène comprenant une étape de mise en contact d'hydroxyde de césium avec du *p*-(benzyloxy)phénol et du paraformaldéhyde, dans lequel ladite base est à une concentration totale supérieure à 0,2 équivalent et inférieure ou égale à 0,5 équivalent, en particulier de 0,3 équivalent, pour obtenir un mélange comprenant du *p*-(benzyloxy)calix[6]arène et du *p*-(benzyloxy)calix[7]arène sous forme salifiée, tel que défini ci-dessus,
comprenant une étape additionnelle de neutralisation du mélange comprenant du *p-*(benzyloxy)calix[6]arène et du *p*-(benzyloxy)calix[7]arène obtenus sous forme salifiée pour obtenir un mélange comprenant du *p*-(benzyloxy)calix[6]arène et du *p*-(benzyloxy)calix[7]arène sous forme neutralisée.

Dans un mode de réalisation avantageux, la présente invention concerne un procédé de préparation d'un composé consistant en un mélange comprenant du *p*-(benzyloxy)calix[6]arène, du *p*-(benzyloxy)calix[7]arène, ou d'un composé consistant en du *p*-(benzyloxy)calix[6]arène ou du *p*-(benzyloxy)calix[7]arène comprenant une étape de mise en contact d'hydroxyde de césium avec du *p*-(benzyloxy)phénol et du paraformaldéhyde, dans lequel ladite base est à une concentration totale supérieure à 0,2 équivalent et inférieure ou égale à 0,5 équivalent, en particulier de 0,3 équivalent, pour obtenir un mélange comprenant du *p*-(benzyloxy)calix[6]arène et du *p*-(benzyloxy)calix[7]arène sous forme salifiée, comprenant une étape additionnelle de neutralisation du mélange comprenant du *p*-(benzyloxy)calix[6]arène et du *p-*(benzyloxy)calix[7]arène obtenu sous forme salifiée pour obtenir un mélange comprenant *du p-*(benzyloxy)calix[6]arène et du *p*-(benzyloxy)calix[7]arène sous forme neutralisée, tel que défini ci-dessus,
comprenant une étape additionnelle de cristallisation du mélange comprenant du *p-*(benzyloxy)calix[6]arène et du *p*-(benzyloxy)calix[7]arène sous forme neutralisée dans un mélange de solvants à base de DMSO ou de DMF pour obtenir le *p*-(benzyloxy)calix[6]arène sous forme neutralisée.

Les Inventeurs ont également trouvé que le traitement du mélange comprenant du *p-*(benzyloxy)calix[6]arène et *p*-(benzyloxy)calix[7]arène sous forme neutralisée dans un mélange de solvants à base de DMSO ou de DMF permettait d'isoler le *p*-(benzyloxy)calix[6]arène pur avec un excellent rendement, supérieur à 50% et une très haute pureté (95%), contrairement à l'art antérieur.

Par l'expression « solvant à base de DMSO ou de DMF », il faut comprendre un mélange de solvant comprenant au moins 10% en volume de DMSO ou de DMF.

Le DMSO ou le DMF permettent de solubiliser le colloïde formé par le mélange comprenant du *p*-(benzyloxy)calix[6]arène et du *p*-(benzyloxy)calix[7]arène sous forme neutralisée.

Dans un mode de réalisation avantageux, la présente invention concerne un procédé de préparation d'un composé consistant en un mélange comprenant du *p*-(benzyloxy)calix[6]arène et du *p*-(benzyloxy)calix[7]arène, ou d'un composé consistant en du *p*-(benzyloxy)calix[6]arène ou du *p*-(benzyloxy)calix[7]arène tel que défini ci-dessus, comprenant les étapes suivantes :
a. mise en contact de l'hydroxyde de césium à une concentration totale supérieure à 0,2 équivalent et inférieure ou égale à 0,5 équivalent, en particulier de 0,3 équivalent, avec du *p*-(benzyloxy)phénol et du formaldéhyde, dans un solvant puis chauffage pour obtenir un mélange comprenant du *p*-(benzyloxy)calix[6]arène et du *p*-(benzyloxy)calix[7]arène sous forme salifiée,
b. neutralisation dudit mélange comprenant du *p*-(benzyloxy)calix[6]arène et du *p-*(benzyloxy)calix[7]arène sous forme salifiée par un acide pour obtenir un mélange comprenant du *p*-(benzyloxy)calix[6]arène et du *p*-(benzyloxy)calix[7]arène sous forme neutralisée,
c. optionnellement, cristallisation du mélange comprenant du *p-*(benzyloxy)calix[6]arène et du *p*-(benzyloxy)calix[7]arène sous forme neutralisée dans un mélange de solvants à base de DMSO ou de DMF pour obtenir le *p-*(benzyloxy)calix[6]arène sous forme neutralisée.

Le solvant de l'étape a. est un solvant possédant un point d'ébullition élevé compris d'environ 100 à environ 200°C, en particulier environ 140°C, par exemple le xylène, mais sans être limité à celui-ci.

Le chauffage dépend donc de la nature du solvant et peut être effectué de 30°C à 200°C.

L'étape a. conduit à un mélange de *p*-(benzyloxy)calix[6]arène et de *p-*(benzyloxy)calix[7]arène sous forme salifiée.

La neutralisation du mélange est effectuée par un acide, par exemple de l'acide chlorhydrique aqueux ou de l'acide sulfurique aqueux.

Après cristallisation dans un mélange de solvants à base de DMSO ou de DMF lorsqu'elle a lieu, le mélange comprenant du *p*-(benzyloxy)calix[6]arène et du *p*-(benzyloxy)calix[7]arène sous forme neutralisée est filtré pour obtenir séparément le *p*-(benzyloxy)calix[6]arène sous forme neutralisée (précipité) et le *p*-(benzyloxy)calix[7]arène (filtrat).

Dans un mode de réalisation avantageux, la présente invention concerne un procédé de préparation d'un composé consistant en un mélange comprenant du *p*-(benzyloxy)calix[6]arène et du *p*-(benzyloxy)calix[7]arène, ou d'un composé consistant en du *p*-(benzyloxy)calix[6]arène ou du *p*-(benzyloxy)calix[7]arène, tel que défini ci-dessus, comprenant les étapes suivantes :
a. mise en contact de l'hydroxyde de césium à une concentration totale supérieure à 0,2 équivalent et inférieure ou égale à 0,5 équivalent, en particulier de 0,3 équivalent, avec du *p*-(benzyloxy)phénol et du formaldéhyde, dans un solvant, puis chauffage pour obtenir un mélange comprenant du *p-*(benzyloxy)calix[6]arène et du *p*-(benzyloxy)calix[7]arène sous forme salifiée,
b. neutralisation dudit mélange comprenant du *p*-(benzyloxy)calix[6]arène et *du p-*(benzyloxy)calix[7]arène sous forme salifiée par un acide pour obtenir un mélange comprenant du *p*-(benzyloxy)calix[6]arène et du *p*-(benzyloxy)calix[7]arène sous forme neutralisée,
c. cristallisation dudit mélange comprenant du *p*-(benzyloxy)calix[6]arène et du *p-*(benzyloxy)calix[7]arène sous forme neutralisée dans un mélange de solvants à base de DMSO pour obtenir le *p*-(benzyloxy)calix[6]arène sous forme neutralisée.

Dans un mode de réalisation avantageux, la présente invention concerne un procédé de préparation d'un composé consistant en un mélange comprenant du *p*-(benzyloxy)calix[6]arène et du *p*-(benzyloxy)calix[7]arène, ou d'un composé consistant du *p*-(benzyloxy)calix[6]arène ou du *p*-(benzyloxy)calix[7]arène, comprenant la mise en contact d'hydroxyde de césium à une concentration totale supérieure à 0,2 équivalent et inférieure ou égale à 0,5 équivalent, en particulier de 0,3 équivalent, avec du p-(benzyloxy)phénol et du formaldéhyde, tel que défini ci-dessus,
dans lequel le chauffage est effectué au reflux du solvant notamment du xylène.

Dans un mode de réalisation avantageux, la présente invention concerne un procédé de préparation d'un composé consistant en un mélange comprenant du *p*-(benzyloxy)calix[6]arène et du *p*-(benzyloxy)calix[7]arène, ou d'un composé consistant en du *p*-(benzyloxy)calix[6]arène ou du *p*-(benzyloxy)calix[7]arène comprenant la mise en contact hydroxyde de césium à une concentration totale supérieure à 0,2 équivalent et inférieure ou égale à 0,5 équivalent, en particulier de 0,3 équivalent, avec du *p*-(benzyloxy)phénol et du formaldéhyde, tel que défini ci-dessus,
dans lequel le mélange de solvants de l'étape de cristallisation comprend du DMSO et un solvant moyennement polaire tel que l'acétone.

Par solvant moyennement polaire, il faut comprendre un solvant possédant un moment dipolair proche de celui de l'acétone.

Le DMSO ou le DMF sont mélangés à un solvant moyennement polaire tel que l'acétone dans une proportion de 10 % à 90 % en volume.

Le filtrat de cristallisation contient alors le *p*-(benzyloxy)calix[7]arène sous forme neutralisée.

Selon un autre aspect, la présente décrit un procédé de préparation d'un composé consistant en un mélange comprenant du *p*-(benzyloxy)calix[9-20]arènes, du *p-*(benzyloxy)calix[6]arène, du *p*-(benzyloxy)calix[7]arène et du *p*-(benzyloxy)calix[8]arène, comprenant une étape de mise en contact d'hydroxyde de rubidium avec du *p*-(benzyloxy)phénol et du paraformaldéhyde, dans lequel ladite base est à une concentration totale supérieure à 0,2 équivalent et inférieure ou égale à 0,5 équivalent, en particulier inférieure ou égale à 0,3 équivalent, pour obtenir un mélange comprenant du *p*-(benzyloxy)calix[9-20]arènes, du *p-*(benzyloxy)calix[6]arène, du *p*-(benzyloxy)calix[7]arène et du *p*-(benzyloxy)calix[8]arène sous forme salifiée.

Les Inventeurs ont trouvé que l'utilisation d'hydroxyde de rubidium à une concentration totale supérieure à 0,2 équivalent et inférieure ou égale à 0,3 équivalent permettait l'obtention d'un mélange comprenant environ 50% en poids d'un mélange de *p*-(benzyloxy)calix[6]arène, de *p*-(benzyloxy)calix[7]arène et de *p*-(benzyloxy)calix[8]arène sous forme salifiée, ainsi qu'un mélange de *p*-(benzyloxy)calix[9-20]arènes sous forme salifiée, à environ 50% en poids.

Dans un mode de réalisation avantageux, la concentration totale d'hydroxyde de rubidium est de 0,21 équivalent, ou de 0,22 équivalent, ou de 0,23 équivalent, ou de 0,24 équivalent, ou de 0,25 équivalent, ou de 0,26 équivalent, ou de 0,27 équivalent, ou de 0,28 équivalent, ou de 0,29 équivalent.

Dans un mode de réalisation avantageux, la présente décrit un procédé de préparation d'un composé consistant en un mélange comprenant du *p*-(benzyloxy)calix[9-20]arènes, du *p*-(benzyloxy)calix[6] arène, du *p*-(benzyloxy)calix[7]arène et du *p-*(benzyloxy)calix[8] arène, comprenant une étape de mise en contact d'hydroxyde de rubidium avec du *p*-(benzyloxy)phénol et du paraformaldéhyde, dans lequel ladite base est à une concentration totale supérieure à 0,2 équivalent et inférieure ou égale à 0,5 équivalent, en particulier inférieure ou égale à 0,3 équivalent, pour obtenir un mélange comprenant du *p-*(benzyloxy)calix[9-20]arènes, du *p*-(benzyloxy)calix[6]arène, du *p*-(benzyloxy)calix[7]arène et du *p*-(benzyloxy)calix[8]arène, sous forme salifiée, tel que défini ci-dessus,
comprenant une étape additionnelle de neutralisation du mélange comprenant du *p-*(benzyloxy)calix[9-20]arènes, du *p*-(benzyloxy)calix[6]arène, du *p*-(benzyloxy)calix[7]arène et du *p*-(benzyloxy)calix[8]arène, sous forme neutralisée.

Dans un mode de réalisation avantageux, la présente décrit un procédé de préparation d'un composé consistant en un mélange comprenant du *p*-(benzyloxy)calix[9-20]arènes, du *p*-(benzyloxy)calix[6] arène, du *p*-(benzyloxy)calix[7]arène et du *p-*(benzyloxy)calix[8] arène, comprenant une étape de mise en contact d'hydroxyde de rubidium avec du *p*-(benzyloxy)phénol et du paraformaldéhyde, dans lequel ladite base est à une concentration totale supérieure à 0,2 équivalent et inférieure ou égale à 0,5 équivalent, en particulier inférieure ou égale à 0,3 équivalent, pour obtenir un mélange comprenant du *p-*(benzyloxy)calix[9-20]arènes, du *p*-(benzyloxy)calix[6]arène, du *p*-(benzyloxy)calix[7]arène et du *p*-(benzyloxy)calix[8]arène, sous forme salifiée, comprenant une étape additionnelle de neutralisation du mélange comprenant du *p*-(benzyloxy)calix[9-20]arènes, du *p-*(benzyloxy)calix[6]arène, du *p*-(benzyloxy)calix[7]arène et du *p*-(benzyloxy)calix[8]arène obtenu sous forme salifiée pour obtenir un mélange comprenant du *p*-(benzyloxy)calix[9-20]arènes, du *p*-(benzyloxy)calix[6]arène, du *p*-(benzyloxy)calix[7]arène et du *p*-(benzyloxy)calix[8]arène, sous forme neutralisée, tel que défini ci-dessus, comprenant une étape additionnelle de cristallisation du mélange comprenant du *p-*(benzyloxy)calix[9-20]arènes, du *p*-(benzyloxy)calix[6]arène, du *p*-(benzyloxy)calix[7]arène et du *p*-(benzyloxy)calix[8]arène sous forme neutralisée dans un mélange de solvants à base de DMSO pour obtenir le *p*-(benzyloxy)calix[6]arène sous forme neutralisée.

Les Inventeurs ont également trouvé que le traitement du mélange comprenant du *p-*(benzyloxy)calix[9-20]arènes, du *p*-(benzyloxy)calix[6]arène, du *p*-(benzyloxy)calix[7]arène et du *p*-(benzyloxy)calix[8]arène sous forme neutralisée dans un mélange de solvants à base de DMSO permettait d'isoler le *p*-(benzyloxy)calix[6]arène, le *p*-(benzyloxy)calix[7]arène, le *p-*(benzyloxy)calix[8]arène ou un de leurs mélanges, sous forme neutralisée, avec un excellent rendement, supérieur ou égal à 50%, contrairement à l'art antérieur.

Par l'expression « solvant à base de DMSO», il faut comprendre un mélange de solvant comprenant au moins 10% en volume de DMSO.

Le DMSO permet de solubiliser le mélange comprenant du *p*-(benzyloxy)calix[6]arène, du *p*-(benzyloxy)calix[7]arène et du *p*-(benzyloxy)calix[8]arène sous forme neutralisée lorsqu'il est repris dans un solvant.

Dans un mode de réalisation avantageux, la présente décrit un procédé de préparation d'un composé consistant en un mélange comprenant du *p*-(benzyloxy)calix[9-20]arènes, du *p*-(benzyloxy)calix[6] arène, du *p*-(benzyloxy)calix[7]arène et du *p-*(benzyloxy)calix[8] arène, tel que défini ci-dessus, comprenant les étapes suivantes :
a. mise en contact de l'hydroxyde de rubidium à une concentration totale supérieure à 0,2 équivalent et inférieure ou égale à 0,5 équivalent, en particulier de 0,3 équivalent, avec du *p*-(benzyloxy)phénol et du formaldéhyde, dans un solvant puis chauffage pour obtenir un mélange comprenant du *p*-(benzyloxy)calix[9-20]arènes, du *p*-(benzyloxy)calix[6]arène, du *p*-(benzyloxy)calix[7]arène et *du p-*(benzyloxy)calix[8]arène sous forme salifiée,
b. neutralisation dudit mélange comprenant du *p*-(benzyloxy)calix[9-20]arènes, du *p-*(benzyloxy)calix[6]arène, du *p*-(benzyloxy)calix[7]arène et du *p-*(benzyloxy)calix[8]arène sous forme neutralisée,
c. optionnellement, cristallisation du mélange comprenant du *p-*(benzyloxy)calix[6]arène et du *p*-(benzyloxy)calix[7]arène sous forme neutralisée dans un mélange de solvants à base de DMSO pour obtenir le *p-*(benzyloxy)calix[6]arène, le *p*-(benzyloxy)calix[7]arène, ou un de leurs mélanges, sous forme neutralisée.

Le solvant de l'étape a. est un solvant possédant un point d'ébullition élevé compris d'environ 100 à environ 200°C, en particulier environ 140°C, par exemple le xylène, mais sans être limité à celui-ci.

Le chauffage dépend donc de la nature du solvant et peut être effectué de 30°C à 200°C.

L'étape a. conduit à un mélange comprenant du *p*-(benzyloxy)calix[9-20]arènes, du *p-*(benzyloxy)calix[6]arène, du *p*-(benzyloxy)calix[7]arène et du *p*-(benzyloxy)calix[8]arène sous forme salifiée.

La neutralisation du mélange est effectuée par un acide, par exemple de l'acide chlorhydrique aqueux ou de l'acide sulfurique aqueux lorsque les calixarènes sont en solution ou en suspension dans un solvant de type xylène.

Dans un mode de réalisation avantageux, la présente décrit un procédé de préparation d'un composé consistant en un mélange comprenant du *p*-(benzyloxy)calix[9-20]arènes, du *p*-(benzyloxy)calix[6]arène, du *p*-(benzyloxy)calix[7]arène et du *p-*(benzyloxy)calix[8]arène, tel que défini ci-dessus, comprenant les étapes suivantes :
a. mise en contact de l'hydroxyde de rubidium à une concentration totale supérieure à 0,2 équivalent et inférieure ou égale à 0,5 équivalent, en particulier de 0,3 équivalent, avec du *p*-(benzyloxy)phénol et du formaldéhyde, dans un solvant puis chauffage pour obtenir un mélange comprenant du *p*-(benzyloxy)calix[9-20]arènes, du *p*-(benzyloxy)calix[6]arène, du *p*-(benzyloxy)calix[7]arène et du *p-*(benzyloxy)calix[8]arène sous forme salifiée,
   neutralisation dudit mélange comprenant du *p*-(benzyloxy)calix[9-20]arènes, du *p-*(benzyloxy)calix[6] arène, du *p*-(benzyloxy)calix[7]arène et du *p-*(benzyloxy)calix[8]arène sous forme neutralisée,
b. cristallisation dudit mélange comprenant du *p*-(benzyloxy)calix[9-20]arènes, du *p-*(benzyloxy)calix[6] arène, du *p*-(benzyloxy)calix[7]arène et du *p-*(benzyloxy)calix[8]arène sous forme neutralisée dans un mélange de solvants à base de DMSO pour obtenir le *p*-(benzyloxy)calix[6]arène, le *p-*(benzyloxy)calix[7]arène, le *p*-(benzyloxy)calix[8]arène ou un de leurs mélanges, sous forme neutralisée.

Dans un mode de réalisation avantageux, la présente décrit un procédé de préparation d'un composé consistant en un mélange comprenant du *p*-(benzyloxy)calix[9-20]arènes, du *p*-(benzyloxy)calix[6]arène, du *p*-(benzyloxy)calix[7]arène et du *p-*(benzyloxy)calix[8] arène, comprenant la mise en contact d'hydroxyde de rubidium à une concentration totale supérieure à 0,2 équivalent et inférieure ou égale à 0,5 équivalent, en particulier de 0,3 équivalent, avec du p-(benzyloxy)phénol et du formaldéhyde, tel que défini ci-dessus,
dans lequel le chauffage est effectué au reflux du solvant notamment du xylène.

Dans un mode de réalisation avantageux, la présente décrit un procédé de préparation d'un composé consistant en un mélange comprenant du *p*-(benzyloxy)calix[9-20]arènes, du *p*-(benzyloxy)calix[6]arène, du *p*-(benzyloxy)calix[7]arène et du *p-*(benzyloxy)calix[8] arène, comprenant la mise en contact d'hydroxyde de rubidium à une concentration totale supérieure à 0,2 équivalent et inférieure ou égale à 0,3 équivalent, avec du *p-*(benzyloxy)phénol et du formaldéhyde, tel que défini ci-dessus,
dans lequel le chauffage est effectué au reflux du solvant notamment du xylène.

Dans un mode de réalisation avantageux, la présente décrit un procédé de préparation d'un composé consistant en un mélange comprenant du *p*-(benzyloxy)calix[6]arène et du *p*-(benzyloxy)calix[7]arène, ou d'un composé consistant en du *p*-(benzyloxy)calix[6]arène ou du *p*-(benzyloxy)calix[7]arène comprenant la mise en contact avec de l'hydroxyde de rubidium à une concentration totale supérieure à 0,2 équivalent et inférieure ou égale à 0,5 équivalent, en particulier de 0,3 équivalent, avec du *p*-(benzyloxy)phénol et du formaldéhyde, tel que défini ci-dessus,
dans lequel le mélange de solvants de l'étape de cristallisation comprend du DMSO et un solvant moyennement polaire tel que l'acétone.

Par solvant moyennement polaire, il faut comprendre un solvant possédant un moment dipolaire proche de celui de l'acétone.

Le DMSO est mélangé à un solvant moyennement polaire tel que l'acétone dans une proportion de 10 % à 90 % en volume.

Le produit obtenu correspond au *p*-(benzyloxy)calix[6]arène, au *p-*(benzyloxy)calix[7]arène, au *p*-(benzyloxy)calix[8]arène ou à un de leurs mélanges.

Selon un autre aspect, l'invention concerne un composé consistant en du *p-*(benzyloxy)calix[7]arène sous forme de monosel de césium.

Le composé de l'invention peut être obtenu par synthèse ou par salification du *p-*(benzyloxy)calix[7]arène sous forme neutralisée au moyen d'au moins une base telle que l'hydroxyde de césium.

Dans un mode de réalisation avantageux, le *p*-(benzyloxy)calix[7]arène neutre ou sous forme de monosel de césium présente une cavité d'environ 1nm permettant notamment de pouvoir inclure des atomes tels que du césium, en particulier du césium radioactif.

Selon un autre aspect, la présente invention concerne un composé consistant en du *p-*(benzyloxy)calix[7]arène sous forme de monosel de césium susceptible d'être obtenu par le procédé comprenant une étape de mise en contact d'au moins une base en particulier l'hydroxyde de césium avec du *p*-(benzyloxy)phénol et du paraformaldéhyde, dans lequel ladite base est à une concentration totale comprise de 0,09 à 0,2 équivalent, en particulier 0,15 équivalent, tel que défini ci-dessus.

Selon encore un autre aspect, la présente décrit l'utilisation d'un composé consistant en du *p*-(benzyloxy)calix[6]arène, du *p*-(benzyloxy)calix[7]arène, optionnellement sous forme de sel de césium, ou du *p*-(benzyloxy)calix[8]arène, ou d'un mélange de ceux-ci, pour la constitution d'un matériau.

Les calixarènes de l'invention peuvent être utilisés notamment pour :
la constitution d'un matériau composite pour la fabrication de matériaux sensibles et éléments capteurs ou,
pour la fabrication d'un matériau textile utilisable pour la purification des métaux, ou la séparation d'un polluant ou d'un contaminant d'un milieu aqueux ou pour la séparation de l'uranium ou autres métaux lourds dans un milieu aqueux.

Ils peuvent donc être utilisés :
soit seuls,
soit en mélange de deux :
   - *p*-(benzyloxy)calix[7]arène optionnellement sous forme de sel de césium et *p-*(benzyloxy)calix[6] arène,
   - *p*-(benzyloxy)calix[6]arène et *p*-(benzyloxy)calix[8]arène,
   - *p*-(benzyloxy)calix[7]arène optionnellement sous forme de sel de césium et *p-*(benzyloxy)calix[8]arène,
soit en mélange de trois :
   - *p*-(benzyloxy)calix[7]arène optionnellement sous forme de sel de césium, *p-*(benzyloxy)calix[6]arène et *p*-(benzyloxy)calix[8]arène,

Dans un mode de réalisation avantageux, les calixarènes utilisés ci-dessus sont susceptibles d'être obtenus par l'un des procédés définis ci-dessus.

L'invention est illustrée au moyen des exemples suivants :

### EXEMPLES

### Exemple 1 : Obtention d'un composé consistant en du p-(benzyloxy)calix[6]arène ou du p-(benzyloxy)calix[7]arène sous forme de sel de césium ou neutralisée ou d'un mélange comprenant du p-(benzyloxy)calix[6]arène et du p-(benzyloxy)calix[7]arène avec de l'hydroxyde de césium.

### Exemple 1.1 : concentration en hydroxyde de césium de 0,15 équivalent

Une suspension de 50,6g de *p*-(benzyloxy)phénol (0,254 mol), 20g (0,667 mol) de paraformaldehyde (point de fusion : 135°C) dans 700 ml de xylène est mise sous argon dans un ballon tricol de 2 litres équipé d'un agitateur mécanique et d'un récupérateur d'eau type « Dean-Stark ».

La suspension est mise à chauffer sous agitation. Lorsque la température atteint 90°C, on ajoute rapidement à l'aide d'une seringue (et sous balayage d'argon) 7,4 ml (0,0425 mol) d'une solution de CsOH à 50% (en poids) dans l'eau.

La suspension est laissée à reflux pendant 6h, période au cours de laquelle on observe la formation d'un volumineux précipité blanc.

Ce précipité est filtré, lavé au xylène, puis au pentane. m=35g, rendement en *p-*(benzyloxy)calix[7]arène (sous forme de monosel de césium) : 58%
Les caractéristiques spectroscopiques RMN de ce précipité correspondent à celle du monoanion du p-(benzyloxy)calix[7]arène : ¹H RMN (DMSO-d6): (déplacements chimiques, ppm) 7,60> delta >7,20 (multiplet, aromatiques) ; 6,72 (singulet fin, hydroquinone) ; 4,93 (singulet fin, protons benzyliques) ; 3,71 (singulet fin, méthylènes intracycliques).
Spectrométrie de masse (MALDI, matrice DHB) : m/z=1617,41 (M+Cs)⁺.

Un échantillon de 1g de ce précipité est mis en suspension dans 5ml de dichlorométhane, puis neutralisé avec une solution aqueuse concentrée d'HCl, sous forte agitation pendant 24h.

La phase organique est récupérée, puis évaporée à sec au rotovapor.

On récupère 0,91g d'un solide blanc, dont les caractéristiques spectroscopiques sont en parfait accord avec le *p*-(benzyloxy)calix[7]arène neutre :
¹H RMN (DMSO-d6): (déplacements chimiques, ppm) 7,30 (multiplet large, aromatiques) ; 6,62 (singulet fin, hydroquinone) ; 4,84 (singulet fin, protons benzyliques) ; 3,74 (singulet fin, méthylènes intracycliques).

Spectrométrie de masse (MALDI, matrice DHB) : m/z=1507,65 (M+Na)⁺.

La perte de masse observée (90mg) est tout à fait en accord avec celle attendue pour la neutralisation d'un monosel de césium : (*p*-(benzyloxy)calix[7]arène⁻.Cs⁺) → *p-*(benzyloxy)calix[7] arène.

### Exemple 1.2 : concentration en hydroxyde de césium de 0,3 équivalent.

Une suspension de 50,6g de *p*-(benzyloxy)phénol (0,254 mol), 20g (0,667 mol) de paraformaldehyde (point de fusion : 135°C) dans 700 ml de xylène est mise sous argon dans un ballon tricol de 2L équipé d'un agitateur mécanique et d'un récupérateur d'eau type « Dean-Stark ».
La suspension est mise à chauffer sous agitation. Lorsque la température atteint 90°C, on ajoute rapidement à l'aide d'une seringue (et sous balayage d'argon) 14,8 ml (0,085 mol) d'une solution de CsOH à 50% (en poids) dans l'eau.

La suspension est laissée à reflux pendant 5h30. On obtient alors une solution parfaitement limpide orange vif.

On ajoute alors 100ml d'une solution de HCl à 37% dans l'eau, et l'on observe la formation d'un précipité. La suspension est laissée sous forte agitation un WE, puis évaporée à sec à l'évaporateur rotatif.

Après lavage avec 500ml d'eau (élimination des sels et de l'excès d'HCl), le solide est dissout à chaud (130°C) dans 200ml de DMSO. On obtient alors une solution limpide noire, à laquelle sont ajoutés à chaud 2 l d'acétone.

Après retour à température ambiante, cette solution limpide est abandonnée une semaine, pendant laquelle un précipité cristallin de *p*-(benzyloxy)calix[6]arène (18g) se dépose sur les parois du ballon.

Le filtrat issu de cette dernière récupération est évaporé au rotovapor, puis au pistolet chauffant jusqu'à obtention d'un solide noir. Ce solide est lavé à l'acétone, ce qui conduit à la récupération d'un deuxième lot de *p*-(benzyloxy)calix[6]arène (10 grammes).
Masse totale de *p*-(benzyloxy)calix[6]arène: 28g, rendement 51%.
¹H RMN (DMSO-d6): (déplacements chimiques, ppm) 7,30 (multiplet large, aromatiques) ; 6,62 (singulet fin, hydroquinone) ; 4,79 (singulet fin, protons benzyliques) ; 3,72 (singulet fin, méthylènes intracycliques).

Spectrométrie de masse (MALDI, matrice DHB) : m/z=1295,49 (M+Na)⁺

### Exemple 2 (comparatif) :

### Obtention d'un composé consistant en du p-(benzyloxy)calix[6]arène ou du p-(benzyloxy)calix[7]arène sous forme neutralisée ou du p-(benzyloxy)calix[8]arène ou d'un mélange comprenant du p-(benzyloxy)calix[6]arène, du p-(benzyloxy)calix[7]arène et du p-(benzyloxy)calix[8] arène avec de l'hydroxyde de sodium ou de potassium.

### Exemple 2.1 : concentration en hydroxyde de sodium ou de potassium de 0,15 équivalent.

Une suspension de 34,5g de *p*-(benzyloxy)phénol (0,173 mol), 20g (0,667 mol) de paraformaldehyde (point de fusion : 135°C) dans 450ml de xylène est mise sous argon dans un ballon tricol de IL équipé d'un agitateur mécanique et d'un récupérateur d'eau type « Dean-Stark ».
La suspension est mise à chauffer sous agitation. Lorsque la température atteint 95°C, on ajoute rapidement à l'aide d'une seringue (et sous balayage d'argon) une solution de 1,43g (0,03 mol) de KOH dans 6ml d'eau Millipore. On observe l'apparition immédiate d'une coloration jaune.
Le milieu réactionnel est porté au reflux pendant 3h30, période pendant laquelle on observe la formation d'un abondant précipité blanc et d'une solution orange vif.
Après retour à température ambiante, le précipité est récupéré par filtration, lavé avec 100ml de xylène et 300ml de pentane.
L'analyse de ce précipité indique qu'il est constitué de *p*-(benzyloxy)calix[7]arène pur. M = 20g, rendement 54%.
Caractérisations :
¹H RMN (DMSO-d6): (déplacements chimiques, ppm) 7,30 (multiplet large, aromatiques) ; 6,62 (singulet fin, hydroquinone) ; 4,84 (singulet fin, protons benzyliques) ; 3,74 (singulet fin, méthylènes intracycliques).
Spectrométrie de masse (MALDI, matrice DHB) : m/z=1524,62 (M+K)⁺.

### Exemple 2.2 : concentration en hydroxyde de sodium ou de potassium de 0,3 équivalent.

Une suspension de 51,5g de *p*-(benzyloxy)phénol (0,258 mol), 20g (0,667 mol) de paraformaldehyde (point de fusion : 135°C) dans 700 ml de xylène est mise sous argon dans un ballon tricol de 2L équipé d'un agitateur mécanique et d'un récupérateur d'eau type « Dean-Stark ».

La suspension est mise à chauffer sous agitation. Lorsque la température atteint 90°C, on ajoute rapidement à l'aide d'une seringue (et sous balayage d'argon) 3,056g de NaOH (0,0764 mol) dans 10 ml d'eau.

La suspension est laissée à reflux pendant 4h30, période au bout de laquelle le milieu réactionnel prend en masse.

Après retour à température ambiante, le milieu réactionnel est neutralisé par 500ml d'une solution d'HCl 2M, sous très forte agitation.

L'émulsion résultante est évaporée à sec et lavée avec 500ml d'eau (élimination des sels de sodium).

Le solide orangé résultant est lavé avec 500ml de THF et le précipité blanc résultant est filtré, ce qui conduit à la récupération de 20g de *p*-(benzyloxy)calix[8]arène pur. Rendement : 36,6%.

¹H RMN (DMSO-d6): (déplacements chimiques, ppm) 7,30 (multiplet large, aromatiques) ; 6,58 (singulet fin, hydroquinone) ; 4,80 (singulet fin, protons benzyliques) ; 3,77 (singulet fin, méthylènes intracycliques).

Spectrométrie de masse (MALDI, matrice DHB) : m/z=1719,62 (M+Na)⁺.

Le filtrat correspondant est évaporé à sec, et dissout à chaud dans 45 ml de DMSO, ce qui conduit à la formation d'une solution homogène noire. IL de toluène y est ajouté, et la solution limpide orange foncée est placée au congélateur (-23°C) pendant 1 semaine, ce qui conduit à la formation d'un précipité microcristallin. Ce précipité est filtré, et son analyse par ¹H RMN montre qu'il s'agit de *p*-(benzyloxy)calix[6]arène pur. M=6,3g, 11%.

¹H RMN (DMSO-d6): (déplacements chimiques, ppm) 7,30 (multiplet large, aromatiques) ; 6,62 (singulet fin, hydroquinone) ; 4,79 (singulet fin, protons benzyliques) ; 3,72 (singulet fin, méthylènes intracycliques).

Spectrométrie de masse (MALDI, matrice DHB) : m/z=1295,49 (M+Na)⁺.

Le filtrat DMSO/toluène correspondant est évaporé jusqu'à obtention d'un liquide orangé. Après ajout de 11 de méthanol et filtration, on récupère 28,5g de *p*-(benzyloxy)calix[7]arène pur. Rendement : 52%.

¹H RMN (DMSO-d6): (déplacements chimiques, ppm) 7,30 (multiplet large, aromatiques) ; 6,62 (singulet fin, hydroquinone) ; 4,84 (singulet fin, protons benzyliques) ; 3,74 (singulet fin, méthylènes intracycliques).
Spectrométrie de masse (MALDI, matrice DHB) : m/z=1524,62 (M+K)⁺.

### Exemple 3 (comparatif) :

### Obtention d'un composé consistant en un mélange comprenant du p-(benzyloxy)calix[6]arène, du p-(benzyloxy)calix[7]arène, du p-(benzyloxy)calix[8]arène et un mélange de p-(benzyloxy)calix[9-20]arènes avec de l'hydroxyde de rubidium.

Dans un ballon de 21 muni d'un agitateur magnétique et d'un Dean-Stark, on prépare une suspension de 50g de 4-(benzyloxy)phénol (0,25 équivalents), 15g de paraformaldehyde (0,5 équivalents) dans 670ml de xylène (mélange d'isomères).

Cette suspension est mise en chauffe. A 90°C, on ajoute rapidement 9 ml (0,3 équivalent par rapport au phénol) d'une solution de RbOH à 50% en poids dans l'eau.

La solution est portée à reflux pendant 6h. On laisse revenir à température ambiante.

La suspension est neutralisée avec 500ml de THF contenant 30ml d'une solution d'HCl à 37% sous forte agitation.

La suspension fluide résultante est filtrée, et le filtrat évaporé à sec. Le solide obtenu est lavé avec 500ml d'acétonitrile et filtré (pour éliminer les espèces les plus polaires, telles que les restes d'oligomères linéaires, le calix[5]arène et l'homooxacalixarène).

Après filtration et séchage sous pompe à vide, on récupère 32 g d'un solide brun, qui est dissout à chaud dans un mélange de 75ml de DMSO et 750ml d'acétone, et immédiatement filtré (récupération du *p*-(benzyloxy)calix[8]arène).

La solution limpide orange foncé résultante est laissée au réfrigérateur pendant deux jours, durée pendant laquelle se forme un précipité microcristallin (20g).

Une étude RMN montre que ce précipité est constitué exclusivement de grands calixarènes.

¹H RMN (DMSO-d6): (déplacements chimiques, ppm) 7,30 (multiplet large, aromatiques) ; 6,52 (singulet fin, hydroquinone) ; 4,77 (singulet fin, protons benzyliques) ; 3,84 (singulet large, méthylènes intracycliques).

Spectrométrie de masse MALDI (matrice DHB) :
2568,97, *p*-(benzyloxy)calix[12]arène.Na ; 2782,07 *p*-(benzyloxy)calix[13]arène.Na ; 2994,14, *p-*(benzyloxy)calix[14]arène.Na ; 3206,24, *p*-(benzyloxy)calix[15]arène.Na ; 3418,31, *p-*(benzyloxy)calix[16]arène.Na.

Le filtrat de récupération du précipité précédent est évaporé d'abord au rotovapor (élimination de l'acétone), puis à la pompe à vide (élimination du DMSO) à 60°C. Lorsque le concentrat DMSO commence à se solidifier, le pompage est arrêté, et le solide très visqueux obtenu est lavé avec 100ml d'acétone, et filtré.

On obtient 15g de *p*-(benzyloxy)calix[6]arène. Rendement : 30%.

## Revendications

1. Procédé de préparation d'un composé consistant en un mélange comprenant du *p-*(benzyloxy)calix[6]arène et du *p*-(benzyloxy)calix[7]arène, ou d'un composé consistant en du *p*-(benzyloxy)calix[6]arène ou du *p*-(benzyloxy)calix[7]arène comprenant une étape de mise en contact d'hydroxyde de césium avec du *p*-(benzyloxy)phénol et du paraformaldéhyde, ledit hydroxyde de césium étant à une concentration totale de 0,09 équivalent à 0,5 équivalent par rapport audit *p*-(benzyloxy)phénol.

2. Procédé selon la revendication 1, dans lequel ledit hydroxyde de césium est à une concentration totale comprise de 0,09 à 0,2 équivalent, en particulier 0,15 équivalent pour obtenir un mélange comprenant du *p*-(benzyloxy)calix[6]arène et du *p*-(benzyloxy)calix[7]arène sous forme salifiée.

3. Procédé selon la revendication 2, comprenant une étape additionnelle de filtration du mélange comprenant du *p*-(benzyloxy)calix[6]arène et du *p*-(benzyloxy)calix[7]arène sous forme salifiée, pour obtenir le *p*-(benzyloxy)calix[7]arène sous forme de monosel de césium suivie d'une étape additionnelle de neutralisation du *p*-(benzyloxy)calix[7]arène sous forme de monosel de césium pour obtenir le *p*-(benzyloxy)calix[7]arène sous forme neutralisée.

4. Procédé selon la revendication 3, comprenant les étapes suivantes :
a. mise en contact de l'hydroxyde de césium à une concentration de 0,09 à 0,2 équivalent, en particulier 0,15 équivalent, avec du *p*-(benzyloxy)phénol et du paraformaldéhyde dans un solvant puis chauffage, pour obtenir un mélange comprenant du *p*-(benzyloxy)calix[6]arène et du *p*-(benzyloxy)calix[7]arène sous forme salifiée,
b. neutralisation du mélange comprenant du *p*-(benzyloxy)calix[6]arène et du *p-*(benzyloxy)calix[7]arène sous forme salifiée avec un acide, pour obtenir un mélange comprenant du *p*-(benzyloxy)calix[6]arène et du *p-*(benzyloxy)calix[7]arène sous forme neutralisée.

5. Procédé selon la revendication 1, dans lequel ladite base est à une concentration totale supérieure à 0,2 équivalent et inférieure ou égale à 0,5 équivalent, en particulier de 0,3 équivalent, pour obtenir un mélange comprenant du *p*-(benzyloxy)calix[6]arène et du *p-*(benzyloxy)calix[7]arène sous forme salifiée.

6. Procédé selon la revendication 5, comprenant une étape additionnelle de neutralisation du mélange comprenant du *p*-(benzyloxy)calix[6]arène et du *p*-(benzyloxy)calix[7]arène obtenu sous forme salifiée pour obtenir un mélange comprenant du *p-*(benzyloxy)calix[6]arène et du *p*-(benzyloxy)calix[7]arène sous forme neutralisée.

7. Procédé selon la revendication 6, comprenant une étape additionnelle de cristallisation du mélange comprenant du *p*-(benzyloxy)calix[6]arène et du *p*-(benzyloxy)calix[7]arène sous forme neutralisée dans un mélange de solvants à base de DMSO ou de DMF pour obtenir le *p*-(benzyloxy)calix[6]arène sous forme neutralisée.

8. Procédé selon l'une des revendications 5 à 7, comprenant les étapes suivantes :
a. mise en contact de l'hydroxyde de césium à une concentration totale supérieure à 0,2 équivalent et inférieure ou égale à 0,5 équivalent, en particulier de 0,3 équivalent, avec du *p*-(benzyloxy)phénol et du formaldéhyde, dans un solvant puis chauffage pour obtenir un mélange comprenant du *p*-(benzyloxy)calix[6]arène et de *p*-(benzyloxy)calix[7]arène sous forme salifiée,
b. neutralisation dudit mélange comprenant du *p*-(benzyloxy)calix[6]arène et du *p-*(benzyloxy)calix[7]arène sous forme salifiée par un acide pour obtenir un mélange comprenant du *p*-(benzyloxy)calix[6]arène et du *p*-(benzyloxy)calix[7]arène sous forme neutralisée,
c. optionnellement, cristallisation du mélange comprenant du *p-*(benzyloxy)calix[6]arène et du *p*-(benzyloxy)calix[7]arène sous forme neutralisée dans un mélange de solvants à base de DMSO ou de DMF pour obtenir le *p-*(benzyloxy)calix[6]arène sous forme neutralisée.

9. Composé consistant en du *p*-(benzyloxy)calix[7]arène sous forme de monosel de césium.

## Patentansprüche

1. Verfahren zur Herstellung einer Verbindung, bestehend aus einer Mischung, umfassend p-(Benzyloxy)-calix[6]aren und p-(Benzyloxy)-calix[7]aren, oder einer Verbindung, bestehend aus p-(Benzyloxy)-calix[6]aren oder p-(Benzyloxy)-calix[7]aren, umfassend einen Schritt des Inberührungbringens von Cesiumhydroxid mit p-(Benzyloxy)-phenol und Paraformaldehyd, wobei das Cesiumhydroxid eine Gesamtkonzentration von 0,09 Äquivalenten bis 0,5 Äquivalenten im Verhältnis zu dem p-(Benzyloxy)-phenol aufweist.

2. Verfahren nach Anspruch 1, wobei das Cesiumhydroxid eine Gesamtkonzentration von 0,09 Äquivalenten bis 0,2 Äquivalenten, insbesondere 0,15 Äquivalenten, aufweist, um eine Mischung zu erhalten, die p-(Benzyloxy)-calix[6]aren und p-(Benzyloxy)-calix[7]aren in versalzter Form umfasst.

3. Verfahren nach Anspruch 2, umfassend einen zusätzlichen Schritt der Filtration der Mischung, die p-(Benzyloxy)-calix[6]aren und p-(Benzyloxy)-calix[7]aren in versalzter Form umfasst, um p-(Benzyloxy)-calix[7]aren in der Form des Cesiummonosalzes zu erhalten, gefolgt von einem zusätzlichen Schritt der Neutralisation von p-(Benzyloxy)-calix[7]aren in der Form des Cesiummonosalzes, um p-(Benzyloxy)-calix[7]aren in neutralisierter Form zu erhalten.

4. Verfahren nach Anspruch 3, umfassend die folgenden Schritte:
a. Inberührungbringen von Cesiumhydroxid mit einer Konzentration von 0,09 bis 0,2 Äquivalenten, insbesondere 0,15 Äquivalenten, mit p-(Benzyloxy)-phenol und Paraformaldehyd in einem Lösungsmittel, anschließendes Erhitzen, um eine Mischung zu erhalten, die p-(Benzyloxy)-calix[6]aren und p-(Benzyloxy)-calix[7]aren in versalzter Form umfasst,
b. Neutralisation der Mischung, die p-(Benzyloxy)-calix[6]aren und p-(Benzyloxy)-calix[7]aren in versalzter Form umfasst, mit einer Säure, um eine Mischung zu erhalten, die p-(Benzyloxy)-calix[6]aren und p-(Benzyloxy)-calix[7]aren in neutralisierter Form umfasst.

5. Verfahren nach Anspruch 1, wobei die Base eine Gesamtkonzentration von größer als 0,2 Äquivalenten und kleiner als oder gleich 0,5 Äquivalenten, insbesondere von 0,3 Äquivalenten, aufweist, um eine Mischung zu erhalten, die p-(Benzyloxy)-calix[6]aren und p-(Benzyloxy)-calix[7]aren in versalzter Form umfasst.

6. Verfahren nach Anspruch 5, umfassend einen zusätzlichen Schritt der Neutralisation der Mischung, die p-(Benzyloxy)-calix[6]aren und p-(Benzyloxy)-calix[7]aren in versalzter Form umfasst, um eine Mischung zu erhalten, die p-(Benzyloxy)-calix[6]aren und p-(Benzyloxy)-calix[7]aren in neutralisierter Form umfasst.

7. Verfahren nach Anspruch 6, umfassend einen zusätzlichen Schritt der Kristallisation der Mischung, die p-(Benzyloxy)-calix[6]aren und p-(Benzyloxy)-calix[7]aren in neutralisierter Form umfasst, in einer Mischung von Lösungsmitteln auf der Basis von DMSO oder von DMF, um p-(Benzyloxy)-calix[6]aren in neutralisierter Form zu erhalten.

8. Verfahren nach einem der Ansprüche 5 bis 7, umfassend die folgenden Schritte:
a. Inberührungbringen von Cesiumhydroxid mit einer Konzentration von größer als 0,2 Äquivalenten und kleiner als oder gleich 0,5 Äquivalenten, insbesondere von 0,3 Äquivalenten, mit p-(Benzyloxy)-phenol und Formaldehyd in einem Lösungsmittel, anschließendes Erhitzen, um eine Mischung zu erhalten, die p-(Benzyloxy)-calix[6]aren und p-(Benzyloxy)-calix[7]aren in versalzter Form umfasst,
b. Neutralisation der Mischung, die p-(Benzyloxy)-calix[6]aren und p-(Benzyloxy)-calix[7]aren in versalzter Form umfasst, mit einer Säure, um eine Mischung zu erhalten, die p-(Benzyloxy)-calix[6]aren und p-(Benzyloxy)-calix[7]aren in neutralisierter Form umfasst,
c. gegebenenfalls Kristallisation der Mischung, die p-(Benzyloxy)-calix[6]aren und p-(Benzyloxy)-calix[7]aren in neutralisierter Form umfasst, in einer Mischung von Lösungsmitteln auf der Basis von DMSO oder von DMF, um p-(Benzyloxy)-calix[6]aren in neutralisierter Form zu erhalten.

9. Verbindung, bestehend aus p-(Benzyloxy)-calix[7]aren in der Form des Cesiummonosalzes.

## Claims

1. Process for the preparation of a compound consisting of a mixture comprising p-(benzyloxy)calix[6]arene and p-(benzyloxy)calix[7]arene, or of a compound consisting of p-(benzyloxy)calix[6]arene or p-(benzyloxy)calix[7]arene comprising a step of contacting caesium hydroxide with *p*-(benzyloxy)phenol and paraformaldehyde, said caesium hydroxide being at a total concentration of 0.09 equivalent to 0.5 equivalent with respect to said *p*-(benzyloxy)phenol.

2. Process according to claim 1, in which said caesium hydroxide is at a total concentration comprised from 0.09 to 0.2 equivalent, in particular 0.15 equivalent in order to obtain a mixture comprising *p*-(benzyloxy)calix[6]arene and *p*-(benzyloxy)calix[7]arene in the salified form.

3. Process according to claim 2, comprising an additional step of filtration of the mixture comprising *p*-(benzyloxy)calix[6]arene and *p*-(benzyloxy)calix[7]arene in the salified form, in order to obtain *p*-(benzyloxy)calix[7]arene in the form of a caesium monosalt followed by an additional step of neutralization of *p*-(benzyloxy)calix[7]arene in the form of a caesium monosalt in order to obtain *p*-(benzyloxy)calix[7]arene in the neutralized form.

4. Process according to claim 3, comprising the following steps:
a. contacting caesium hydroxide at a concentration from 0.09 to 0.2 equivalent, in particular 0.15 equivalent, with *p*-(benzyloxy)phenol and paraformaldehyde in a solvent, then heating, in order to obtain a mixture comprising *p-*(benzyloxy)calix[6]arene and *p*-(benzyloxy)calix[7]arene in the salified form,
b. neutralization of the mixture comprising *p*-(benzyloxy)calix[6]arene and *p-*(benzyloxy)calix[7]arene in the salified form with an acid, in order to obtain a mixture comprising *p*-(benzyloxy)calix[6]arene and *p*-(benzyloxy)calix[7]arene in the neutralized form.

5. Process according to claim 1, in which said base is at a total concentration greater than 0.2 equivalent and less than or equal to 0.5 equivalent, in particular 0.3 equivalent, in order to obtain a mixture comprising *p*-(benzyloxy)calix[6]arene and *p*-(benzyloxy)calix[7]arene in the salified form.

6. Process according to claim 5, comprising an additional step of neutralization of the mixture comprising *p*-(benzyloxy)calix[6]arene and *p*-(benzyloxy)calix[7]arene obtained in the salified form in order to obtain a mixture comprising *p*-(benzyloxy)calix[6]arene and *p*-(benzyloxy)calix[7]arene in the neutralized form.

7. Process according to claim 6, comprising an additional step of crystallization of the mixture comprising *p*-(benzyloxy)calix[6]arene and *p*-(benzyloxy)calix[7]arene in the neutralized form from a mixture of DMSO- or DMF-based solvents in order to obtain *p-*(benzyloxy)calix[6]arene in the neutralized form.

8. Process according to one of claims 5 to 7, comprising the following steps:
a. contacting caesium hydroxide at a total concentration greater than 0.2 equivalent and less than or equal to 0.5 equivalent, in particular 0.3 equivalent, with *p-*(benzyloxy)phenol and formaldehyde in a solvent, then heating in order to obtain a mixture comprising *p*-(benzyloxy)calix[6]arene and *p*-(benzyloxy)calix[7]arene in the salified form,
b. neutralization of said mixture comprising *p*-(benzyloxy)calix[6]arene and *p-*(benzyloxy)calix[7]arene in the salified form with an acid in order to obtain a mixture comprising *p*-(benzyloxy)calix[6]arene and *p*-(benzyloxy)calix[7]arene in the neutralized form,
c. optionally, crystallization of the mixture comprising *p*-(benzyloxy)calix[6]arene and *p*-(benzyloxy)calix[7]arene in the neutralized form from a mixture of DMSO-or DMF-based solvents in order to obtain *p*-(benzyloxy)calix[6]arene in the neutralized form.

9. Compound consisting of *p*-(benzyloxy)calix[7]arene in the form of a caesium monosalt.
